(19) Europäisches Patentamt
European Patent Office
Office européen des brevets

(11) Veröffentlichungsnummer : **0 014 976**
**B1**

(12) # EUROPÄISCHE PATENTSCHRIFT

(45) Veröffentlichungstag der Patentschrift : **07.04.82**

(51) Int. Cl.$^3$ : **C 07 D203/22**, A 61 K 31/395

(21) Anmeldenummer : **80100792.3**

(22) Anmeldetag : **16.02.80**
**Die Akte enthält technische Angaben, die nach dem Eingang der Anmeldung eingereicht wurden und die nicht in dieser Patentschrift enthalten sind.**

(54) **N-substituierte 2-Cyanaziridine, Verfahren zu deren Herstellung sowie diese Substanzen enthaltende Arzneimittel, entsprechend substituierte Aziridin-2-carbonsäureamide und deren Herstellung.**

(30) Priorität : **21.02.79 DE 2906603**

(43) Veröffentlichungstag der Anmeldung : **03.09.80 (Patentblatt 80/18)**

(45) Bekanntmachung des Hinweises auf die Patenterteilung : **07.04.82 Patentblatt 82/14**

(84) Benannte Vertragsstaaten : **AT BE CH DE FR GB IT LU NL SE**

(56) Entgegenhaltungen :
**DE - A1 - 2 644 820**
**Chemical Abstracts, Band 77, Nr. 25 1972, Columbus, Ohio, USA, CH. SHIN et al. « Reaction of ethyl α, β-unsaturated β-nitrocarboxylates with triethyl phosphite » Seite 429, rechte Spalte, Abstract Nr. 164799h**
**Chemical Abstracts, Band 81, 1974 Columbus, Ohio, USA, R.G. KOSTYANOVSKII et al. : « N-Alkoxyaziridine-2-carboxylates », Seite 416, linke Spalte, Abstract Nr. 25474t**
**Chemical Abstracts, Band 85, 1976 Columbus, Ohio, USA R.G. KOSTYANOVSKII et al. : « Asymmetric non-bridging nitrogen. Communication 9. Derivatives of 1-alkoxyaziridine-2-carboxylic acids » Seite 529, linke Spalte, Abstract Nr. 192460z**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Bosies, Elmar, Dr.rer.nat.**
**Delpstrasse 11**
**D-6940 Weinheim (DE)**
Erfinder : **Kampe, Wolfgang, Dr.rer.nat.**
**Zedernstrasse 49**
**D-6805 Heddesheim (DE)**
Erfinder : **Thiel, Max, Dr.rer.nat.**
**S 6, 35**
**D-6800 Mannheim 1 (DE)**
Erfinder : **Bicker, Uwe, Dr.rer.nat.**
**Hirschstrasse 59**
**D-6143 Lorsch (DE)**
Erfinder : **Boerner, Dietmar, Dr.med.**
**Joh.-Walterstrasse 21**
**D-6840 Lampertheim-Hüttenfeld (DE)**

**EP 0 014 976 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

N-substituierte 2-Cyanaziridine, Verfahren zu deren Herstellung sowie diese Substanzen enthaltende Arzneimittel, entsprechend substituierte Aziridin-2-carbonsäureamide und deren Herstellung

Gegenstand der vorliegenden Erfindung sind neue N-substituierte 2-Cyanaziridine der allgemeinen Formel Ia

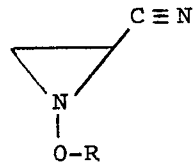

(Ia)

in der

R einen gesättigten $C_1$-$C_8$-Alkylrest, der ein- oder mehrfach durch Halogen, Hydroxy, Carboxyl, Nitril, Nitro oder einen $C_1$-$C_8$-Alkoxy-, $C_1$-$C_8$-Alkoxy-carbonyl-, Carbamoyl-, Formamido-, Acetamido-, Benzamido-, Dimethylamino-, Methylmercapto-, Methylsulfinyl- oder Methylsulfonyl-Rest oder einen $C_3$-$C_{10}$-Cycloalkyl- oder Cycloalkenyl-Rest oder einen Phenoxy-Rest oder einen Phenyl-Rest substituiert sein kann, welcher wiederum gegebenenfalls ein- oder mehrfach duıch Halogen, Hydroxy, Nitro, Trifluormethyl, Nitril, Methylmercapto oder einen $C_1$-$C_8$-Alkyl- oder Alkoxy-Rest substituiert ist,

einen ein- oder mehrfach ungesättigten Alkenyl- oder Alkinyl-Rest mit 3-8 C-Atomen, einen $C_3$-$C_{10}$-Cycloalkyl- oder Cycloalkenyl-Rest, einen Cinnamyl-, Norbornyl- oder Adamantyl-Rest, einen Phenyl- oder Naphthyl-Rest oder einen Tetrahydrofurfuryl-, Tetrahydropyranyl-, Acetoxyäthyl-, Morpholinoethyl-, N-Acetyl-piperidin-4-ylmethyl-, Thianyl-, Pyridinylmethyl-, Furfuryl-, Thenyl-, Pyrimidinylmethyl-, 2-Oxazolidinon-5-ylmethyl- oder 2-Cyan-1-aziridinyloxyethyl-Rest

bedeutet, sowie deren pharmakologisch verträglichen Salze, Verfahren zu deren Herstellung sowie diese Substanzen enthaltende Arzneimittel.

Die Verbindungen der allgemeinen Formel Ia besitzen asymmetrische Kohlenstoffatome und können in cis-trans-Isomeren auftreten. Gegenstand der Erfindung sind sämtliche stereoisomeren Formen sowie deren Gemische. Die gegebenenfalls durchzuführende Trennung der stereoisomeren Formen wird nach an sich bekannten Verfahren vorgenommen.

Aus den deutschen Offenlegungsschriften 27 27 550, 26 56 323 und 27 31 264 sind immunstimulierend wirkende Aziridin-2-carbonsäurederivate bekannt, die am Ringstickstoffatom acyliert sind.

Es wurde nunmehr überraschenderweise gefunden, daß eine Klasse von 2-Cyanaziridinen, die am Ringstickstoffatom anstelle der Acylgruppe einen Alkoxy-, Aryloxy- oder Hetaryloxy-Substituenten tragen, eine deutlich bessere Immunstimulation zeigen und zusätzlich keine nennenswerten Nebenwirkungen aufweisen. Diese Substanzen eignen sich daher vorzüglich zur Bekämpfung von Krankheiten, die mit einer Schwächung des Immunsystems verbunden sind.

Desweiteren wurde im Tierversuch gefunden, daß die erfindungsgemäßen Substanzen eine Wirksamkeit gegen maligne Tumore besitzen und daher als Adjuvans für eine Immuntherapie maligner Tumore geeignet sind. Ferner wurde gefunden, daß diese Substanzen auch in der Lage sind, die Knochenmarkstoxizität von Röntgenbestrahlungen oder Zystostatica zu verringern bzw. teilweise zu kompensieren.

Verbindungen der allgemeinen Formel Ia, die in 2-Stellung anstelle des Nitril-Substituenten eine Alkoxycarbonyl- oder eine Carbamoylgruppe aufweisen, sind zum Teil literaturbekannte Verbindungen (siehe beispielsweise Chem. Abstracts *81,* 25 474 t und *85,* 192 460 z). Angaben über eine pharmakologische Wirksamkeit dieser Verbindungen sind diesen Literaturstellen jedoch nicht zu entnehmen.

Ein weiterer Gegenstand der Erfindung sind Verbindungen der allgemeinen Formel Ib

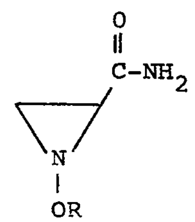

(Ib)

in der R die bei der Formel Ia angegebene Bedeutung hat. Diese Verbindungen sind Zwischenprodukte bei der Herstellung der Verbindungen der allgemeinen Formel Ia.

Die immunstimulierende Wirkung der erfindungsgemäßen Verbindungen kann nachgewiesen werden :

1. durch den Anstieg der Leukozyten nach oraler und intravenöser Applikation der obenerwähnten Substanzen,

2. durch die Erhöhung der Lymphozytentransformation, gemessen mit Hilfe des Einbaus von

radioaktiv-markiertem Thymidin in Humanlymphozyten nach Inkubation mit den obenerwähnten Substanzen (vgl. hierzu : K. Resch in « Praxis der Immunologie », Herausgeber : K.O. Vorländer, Thieme-Verlag. Stuttgart 1976) und

3. mit Hilfe einer tierexperimentellen Infektion bei Mäusen.

Bei letzterer Untersuchung hat es sich überraschenderweise gezeigt, daß die zusätzliche Applikation der Substanzen der allgemeinen Formel Ia zu einem bekannten bakteriostatisch wirkenden Chemotherapeuticum, z.B. Chloramphenicol, einen deutlicheren therapeutischen Effekt zeigt als die alleinige Applikation des bakteriostatischen Chemotherapeutikums.

Gegenstand der Erfindung sind daher ferner Arzneimittel, die zusätzlich zu einer Verbindung der allgemeinen Formel Ia und geeigneten Träger- und Hilfsstoffen ein Chemotherapeuticum enthalten, wobei unter Chemotherapeutica handelsübliche Substanzen mit antimikrobieller Wirkung, z.B. Penicilline, Cephalosporine, Sulfonamide, Aminoglykosid-Antibiotika, Tetracycline u.a. zu verstehen sind. Der synergistische Effekt zeigt sich deutlich beispielsweise bei der oben angesprochenen Arzneimittelkombination, die ein Immunstimulans aus der Gruppe der Verbindungen der allgemeinen Formel Ia und das bakteriostatisch wirkende Chemotherapeuticum Chloramphenicol enthält.

Die in der Definition des Substituenten R vorkommenden Alkylreste mit 1-8, insbesondere 1-6 Kohlenstoffatomen können geradkettige oder verzweigt sein. Vorzugsweise findet die Methyl-, Ethyl-, Propyl-, Isopropyl-, n-Butyl-, sec.-Butyl-, tert.-Butyl-, n-Pentyl- und n-Hexylgruppe Verwendung.

Unter den ein- oder mehrfach ungesättigten Alkylresten mit 3-8, vorzugsweise 3-5 Kohlenstoffatomen sind der Allyl-, Methylallyl-, Crotyl-, 1-Methylprop-2-enyl, Propargyl-, 2-Butinyl-, 1-Methyl-but-2-inyl- und 3-Pentinylrest besonders bevorzugt.

Als Cycloalkyl-bzw. Cycloalkenylreste mit 3-10 Kohlenstoffatomen sind, insbesondere der Cyclopropyl-, Cyclopentyl-, der Cyclohexyl-, der Cyclohexenyl-, und der Cycloheptenylrest zu nennen.

Halogen im Sinne der vorliegenden Erfindung soll Fluor, Chlor und Brom sein.

Die Verbindungen der allgemeinen Formel I (Zusammenfassung von Ia und Ib) lassen sich nach an sich bekannten Verfahren darstellen. Insbesondere sind diese Verfahren dadurch gekennzeichnet, daß man in an sich bekannter Weise

a) eine Verbindung der allgemeinen Formel II

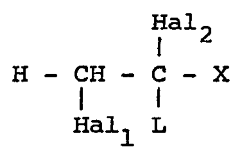

(II)

in der

X entweder $-C\equiv N$ oder $-\overset{O}{\overset{\|}{C}}-NH_2$ bedeutet, $Hal_1$ und $Hal_2$ Chlor oder Brom sind, L Wasserstoff bedeutet, wobei $Hal_1$ und L zusammen auch ein Valenzstrich sein können,
mit einem Hydroxylaminderivat der allgemeinen Formel III

$$R-O-NH_2 \qquad (III)$$

in der

R die oben angegebene Bedeutung hat,
umsetzt oder

b) eine Verbindung der allgemeinen Formel IVa oder IVb bzw. deren Salze

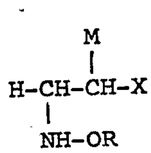

(IVa)

bzw.

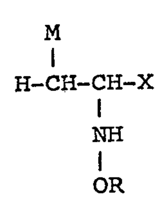

(IVb),

in denen

R und X die oben angegebenen Bedeutungen haben und M Chlor, Brom oder die Gruppe A-Z bedeuten soll, wobei A Sauerstoff oder Schwefel und Z Wasserstoff oder eine zusammen mit Sauerstoff bzw. Schwefel leicht eliminierbare Gruppierung darstellen soll,
mit einem M-H-abspaltenden Reagenz behandelt

oder

c) eine Verbindung der allgemeinen Formel V

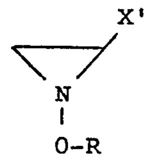

(V)

in der
R die oben angegebene Bedeutung hat und X' die $-CH=NOR_2$-Gruppe darstellt, wobei $R_2$ Wasserstoff oder eine Alkylgruppe vorstellt,
mit einem wasser- bzw. alkoholabspaltenden Reagenz behandelt
oder

d) ein Oxazolidinon der allgemeinen Formel VIa oder VIb

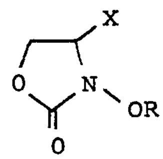
(VIa)

bzw.

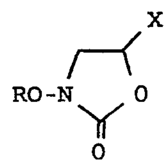
(VIb),

in denen
R und X die oben angegebenen Bedeutungen haben,
einer Thermolyse unterwirft
oder

e) eine Verbindung der allgemeinen Formel VIIa oder VIIb

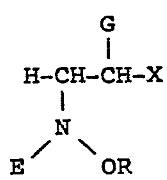
(VIIa)

bzw.

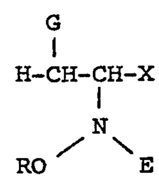
(VIIb),

in denen
R und X die oben angegebenen Bedeutungen haben und G Wasserstoff, Chlor oder Brom und E Chlor, Brom, eine Trialkylaminogruppe oder einen Arylsulfonsäureresterrest bedeuten,
mit einem E-G- apspaltenden Reagenz behandelt
oder

f) ein Epoxyd der allgemeinen Formel VIII

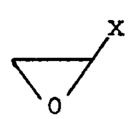

(VIII)

in der
X die oben angegebene Bedeutung hat,
mit einem Hydroxylaminderivat der allgemeinen Formel III umsetzt,
anschließend gewünschtenfalls eine nach einem der vorstehenden Verfahren erhaltene Verbindung der allgemeinen Formel I mit X = Carbamoyl in die entsprechende Nitril-Verbindung überführt oder einen Substituenten R in einen anderen durch den Anspruch definierten Substituenten R umwandelt und gegebenenfalls eine so erhaltene Verbindung der allgemeinen Formel Ia in ein pharmakologisch verträgliches Salz überführt.

Bei Verfahren a) und b) setzt man als Halogenwasserstoffabspaltendes Reagenz Basen ein, vor allem

tert. Amine wie Triethylamin, Triethanolamin, Diazabicycloalkene u.a. Hierbei finden Alkohole wie Methanol, Ethanol und Butanol aber auch inerte Lösungsmittel wie Methylenchlorid, Dioxan, Benzol oder Toluol Verwendung. Sehr gut lassen sich auch Lösungsmittel wie Dimethylformamid oder Dimethylsulfoxyd einsetzen. Darüberhinaus finden in einigen Fällen vor allem Alkoholate wie Natriummethylat oder Natriumethylat in dem entsprechenden Alkohol Anwendung. Bewährt hat sich bei Verfahren b), vor allem, wenn die Gruppe A-Z die OH-Gruppe bedeutet, als wasserabspaltendes Mittel Triphenylphosphin in Gegenwart von Tetrachlorkohlenstoff und Triethylamin, wobei dann in der Regel Methylenchlorid oder Chloroform als Lösungsmittel eingesetzt wird. Die Wasserabspaltung gelingt jedoch auch mit Schwefelsäure.

Bei Verfahren c) kann man die üblichen literaturbekannten Methoden zur Umwandlung einer Oximgruppe in die Nitrilgruppe anwenden, wie z.B. die Wasser- bzw. Alkoholabspaltung mit Hilfe von Thionylchlorid, Phosphorpentachlorid, Phosphorpentoxid, Trifluoracetanhydrid, Dicyclohexylcarbodiimid u.a. Hierbei werden als Hilfsbasen, Amine wie Triethylamin, Pyridin u.a. eingesetzt. Unter sehr schonenden Bedingungen läuft die Umsetzung mit Triphenylphosphin in Gegenwart von Tetrachlorkohlenstoff und Triethylamin ab. Als Lösungsmittel wird hierbei bevorzugt Methylenchlorid oder Chloroform verwendet.

Oxazolidinone der allgemeinen Formel VIa bzw. VIb werden beim Verfahren d) in der Regel ohne Lösungsmittel in Gegenwart von Basen, wie z.B. Triethanolamin oder Dicyclohexylethylamin thermolysiert, wobei das Reaktionsprodukt während der Thermolyse destillativ entfernt wird. Die Thermolysetemperaturen liegen üblicherweise zwischen 170 °C und 250 °C.

Bei Verfahren e) werden als E-G-abspaltende Reagenzien im Falle, daß G gleich Wasserstoff ist, bevorzugt Alkoholate wie Alkalimethylat oder Alkaliethylat in den entsprechenden Alkoholen eingesetzt. Man kann jedoch auch tert. Amine wie Triethylamin, Triethanolamin, Dicyclohexylethylamin oder Diazabicycloundecen in Lösungsmitteln wie Methanol, Ethanol, Benzol, Toluol, Diethylether oder Dioxan verwenden. Für den Fall, daß E und G Chlor oder Brom sind, kann man zur Abspaltung gängige Enthalogenierungsmittel, vorzugsweise Zink oder Natrium, einsetzen.

Bei Verfahren f) kann man ein Epoxid der allgemeinen Formel VIII mit Hydroxylaminderivaten der allgemeinen Formel III reagieren lassen und den dabei entstehenden Aminoalkohol wie bei Verfahren b) beschrieben zu einem Aziridinderivat der allgemeinen Formel I dehydratisieren. Man kann jedoch zur Umwandlung des Epoxids in ein Aziridin auch sehr gut Verbindungen wie R-O-N-P(O) $(OAlK)_2^{\ominus}$ oder $Ph_3P$ = N-OR einsetzen, wobei R die oben angegebene Bedeutung hat, Ph Phenyl und Alk niederes Alkyl wie Methyl oder Ethyl sein soll.

Zur Umwandlung einer nach den Verfahren a, b, d, e oder f erhaltenen Verbindung der allgemeinen Formel Ib in die entsprechende Nitrilverbindung der allgemeinen Formel Ia werden literaturbekannte Dehydratisierungsmittel eingesetzt, wobei vor allem das Gemisch aus Triphenylphosphin, Tetrachlorkohlenstoff und Triethylamin angewendet wird. Als Lösungsmittel nimmt man üblicherweise halogenierte Kohlenwasserstoffe wie z.B. Methylenchlorid bzw. Chloroform oder aber auch Acetonitril. Das gewünschte Nitril wird in der Regel durch Destillation aus dem Reaktionsgemisch isoliert.

Die für die Herstellung der erfindungsgemäßen Verbindungen benötigten Ausgangsprodukte sind bekannte Substanzen oder können in Analogie zu den für die Herstellung der bekannten Verbindungen beschriebenen Methoden dargestellt werden.

Verbindungen der Formel IV a lassen sich beispielsweise durch Anlagerung von O-substituierten Hydroxylaminderivaten an aktivierte, vorzugsweise bromaktivierte Acrylsäurederivate nach üblichen Methoden erhalten. Die Oxime der allgemeinen Formel V lassen sich in bekannter Weise aus den entsprechenden Aldehyden und den Hydroxylaminderivaten erhalten. Zur Herstellung von Verbindungen der Formel VI werden vorzugsweise N-OR-substituierte Serin- bzw. Isoserin-Derivate mit Phosgen, Chlorameisensäureestern u.ä. cyclisiert.

Zur Herstellung pharmazeutischer Mittel mit immunstimulierender Wirkung werden die Verbindungen der allgemeinen Formel Ia in an sich bekannter Weise mit geeigneten pharmazeutischen Traegersubstanzen gemischt und beispielsweise als Tabletten oder Dragees ausgeformt oder unter Zugabe entsprechender Hilfsstoffe in Wasser oder Oel, wie z.B. Olivenoel, suspendiert oder geloest und in Steckkapseln abgefuellt. Da der Wirkstoff saeurelabil ist, wird die Zubereitung mit einem erst im alkalischen Duenndarmmilieu loeslichen Ueberzug versehen oder ein entsprechender Traegerstoff, wie beispielsweise eine hoehere Fettsaeure oder Carboxymethylcellulose, zugemischt. Feste Traegerstoffe sind z.B. Staerke, Lactose, Mannit, Methylcellulose, Talkum, hochdisperse Kieselsaeuren, hoehermolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole), fuer orale Applikation geeignete Zubereitungen koennen gewuenschtenfalls Geschmacks- und Sueßstoffe enthalten.

Als Injektionsmedium kommt vorzugsweise Wasser zur Anwendung, welches die bei Injektionsloesungen ueblichen Zusaetze wie Stabilisierungsmittel, Loesungsvermittler oder schwach alkalische Puffer enthaelt. Derartige Zusaetze sind z.B. Dimethylsulfoxyd, Dimethylformamid, N-Methylpyrrolidon, Phosphat- oder Carbonatpuffer, Ethanol, Komplexbildner (wie Ethylendiamintetraessigsaeure und deren nicht-toxische Salze), hochmolekulare Polymere (wie fluessiges Polyethylenoxyd) zur Viskositaetsregulierung.

Zur Bekämpfung von Krankheiten, die mit einer Schwächung des Immunsystems verbunden sind,

werden die pharmakologisch aktiven Verbindungen der allgemeinen Formel Ia in Einzeldosen von 1 bis 600, vorzugsweise von 50 bis 500 mg angewandt, wobei diese Einzeldosen je nach Bedarf ein- oder mehrmals pro Tag verabreicht werden können.

Für Arzneimittelkombinationen, in denen die Verbindungen der allgemeinen Formel Ia zusammen mit einem Chemotherapeutikum vorliegen, kommen im allgemeinen die gleichen galenischen Zubereitungsformen in Betracht wie für die oben aufgeführten Einzelsubstanzen. Die beiden Wirkstoffe, ein Immunstimulans sowie das Chemotherapeuticum, liegen in der Regel in der Zubereitungsform im Verhältnis 10 : 1 bis 1 : 10 vor, wobei sich als vorteilhaft ein äquimolares Verhältnis der beiden Komponenten erwiesen hat.

Eine geeignete Zubereitung besteht z.B. aus 100 mg Chloramphenicol als Chemotherapeutikum und 33,3 mg 2-Cyan-1-ethoxyaziridin sowie geeigneten Trägerstoffen wie Stärke und wird in Form von 250 mg Tabletten hergestellt, die in der Regel zweimal pro Tag oral eingenommen werden.

Bevorzugt im Sinne der vorliegenden Anmeldung sind ausser den in den folgenden Beispielen genannten Verbindungen noch die folgenden :

2-Cyan-1-n-propoxyaziridin
1-sec.-Butoxy-2-cyanaziridin
1-tert.-Butoxy-2-cyanaziridin
2-Cyan-1-pentyloxyaziridin
2-Cyan-1-hexyloxyaziridin
1-(3-Chlorpropoxy)-2-cyanaziridin
2-Cyan-1-(2.2.2.-trichlorethoxy)-aziridin
2-Cyan-1-(2-fluorethoxy)-aziridin
2-Cyan-1-(2-hydroxyethoxy)-aziridin
2-Cyan-1-(2.3-dihydroxypropoxy)-aziridin
2-Cyan-1-(2-methoxyethoxy)-aziridin
2-Cyan-1-tetrahydrofurfuryloxyaziridin
2-Cyan-1-(tetrahydropyran-2-yloxy)-aziridin
2-Cyan-1-(2-phenoxyethoxy)-aziridin
1-(2-Acetoxyethoxy)-2-cyanaziridin
2-Cyan-1-(3-N.N-dimethylaminopropoxy)-aziridin
1,2-Bis-(2-cyan-1-aziridinyloxy)-ethan
2-Cyan-1-(-2-morpholinoethoxy)-aziridin
1-(2-Acetamidoethoxy)-2-cyanaziridin
1-(2-Benzamidoethoxy)-2-cyanaziridin
1-(1-Acetylpiperidin-4-ylmethoxy)-2-cyanaziridin
2-Cyan-1-(2-oxazolidinon-5-ylmethoxy)-aziridin
2-Cyan-1-(2-nitroethoxy)-aziridin
2-Cyan-1-(3-methylmercaptopropoxy)-aziridin
2-Cyan-1-(2-methylsulfinylethoxy)-aziridin
2-Cyan-1-(2-methylsulfonylethoxy)-aziridin

2-Cyan-1-(thian-3-yloxy)-aziridin

2-Cyan-1-(2-cyanethoxy)-aziridin

2-Cyanaziridin-1-yl-oxyessigsäure
2-Cyanaziridin-1-yl-oxyessigsäureethylester
2-Cyanaziridin-1-yl-oxyessigsäureamid
2-(2-Cyanaziridin-1-yl-oxy)-propionsäuremethylester
2-Cyan-1-methallyloxyaziridin
2-Cyan-1-(1-methyl-prop-2-enyloxy)-aziridin
1-(But-2-enyloxy)-2-cyanaziridin
1-Cinnamyloxy-2-cyanaziridin
2-Cyan-1-propargyloxyaziridin
1-(But-2-inyloxy)-2-cyanazirdin
2-Cyan-1-(1-methyl-prop-2-inyloxy)-aziridin
2-Cyan-1-(pent-3-inyloxy)-aziridin
2-Cyan-1-cyclopropylmethoxy-aziridin
2-Cyan-1-(cyclohex-3-enylmethoxy)-aziridin
2-Cyan-1-cyclohexyloxyaziridin

2-Cyan-1-norbornyloxyaziridin
2-Cyan-1-phenoxyaziridin
2-Cyan-1-(3-trifluormethylbenzyloxy)-aziridin

2-Cyan-1-(3-fluorbenzyloxy)-aziridin
2-Cyan-1-(4-cyanbenzyloxy)-aziridin
1-(4-tert.-Butylbenzyloxy)-2-cyanaziridin
2-Cyan-1-(4-nitrobenzyloxy)-aziridin

2-Cyan-1-(2-methylmercaptobenzyloxy)-aziridin
2-Cyan-1-(3,4-dichlorbenzyloxy)-aziridin
2-Cyan-1-(3,4-methylendioxybenzyloxy)-aziridin
2-Cyan-1-(2-methoxy-4-nitrobenzyloxy)-aziridin
2-Cyan-1-(2-hydroxy-5-nitrobenzyloxy)-aziridin
2-Cyan-1-(3.4.5-trimethoxybenzyloxy)-aziridin

1-Benzhydryloxy-2-cyanaziridin
2-Cyan-1-(2-naphthyloxy)-aziridin
2-Cyan-1-(2-furylmethoxy)-aziridin
2-Cyan-1-(3-thenyloxy)-aziridin
2-Cyan-1-(2-pyridylmethoxy)-aziridin
2-Cyan-1-(2-pyrimidinylmethoxy)-aziridin

D-(+)-2-Cyan-1-(L-(−)-1-phenylethoxy)-aziridin
D-(+)-2-Cyan-1-(D-(+)-1-phenylethoxy)-aziridin
L-(−)-2-Cyan-1-(L-(−)-1-phenylethoxy)-aziridin
L-(−)-2-Cyan-1-(D-(+)-1-phenylethoxy)-aziridin

Die nachfolgenden Beispiele zeigen einige der zahlreichen Verfahrensvarianten, die zur Synthese der erfindungsgemäßen Verbindungen verwendet werden können. Sie sollen jedoch keine Einschränkung des Erfindungsgegenstandes darstellen.

Die Struktur der in den nachfolgenden Beispielen beschriebenen Substanzen ist durch Mikroverbrennungsanalyse, NMR- und Massenspektrum gesichert.

## Beispiel 1

2-Cyan-1-ethoxyaziridin

Zu 2.29 g 2-Brom-3-ethoxyaminopropionitril-hydrochlorid (Fp : 97-100 °C) in 30 ml Toluol gibt man 2.98 g Triethanolamin und läßt 5 h unter Rückfluß kochen. Das Toluol wird dann am Rotationsverdampfer abgezogen, der Rückstand mit Ether behandelt, filtriert und das Filtrat eingeengt. Den Rückstand nimmt man in wenig eiskalter 2 N Salzsäure auf, extrahiert dreimal mit Ether, wäscht die Etherphase mit Wasser neutral, trocknet über Natriumsulfat und engt ein. Der Rückstand wird dann destilliert.

Ausbeute : 0.6 g (≙ 53 % d. Th.) 2-Cyan-1-ethoxyaziridin vom $Kp_{0,1}$ : 38-40 °C

In analoger Weise erhält man aus

a) 3-Benzyloxyamino-2-brompropionitril-hydrochlorid (Fp : 128-130 °C)
das 1-Benzyloxy-2-cyanaziridin ($Kp_{0,1}$ : 113-115 °C)

b) 2-Brom-3-isopropoxyaminopropionitril-hydrochlorid (Fp : 100-104 °C)
das 2-Cyan-1-isopropoxyaziridin ($Kp_{0,1}$ : 28-29 °C)

## Beispiel 2

2-Cyan-1-ethoxyaziridin

Zu 4.7 g 2.3-Dibrompropionitril in 10 ml Ethanol tropft man unter Rühren bei 20 °C eine Lösung von 3.3 g Triethanolamin in 10 ml Ethanol zu. Nach 1 h tropft man eine Lösung von 1.8 g O-Ethylhydrooxylamin in 10 ml Ethanol und eine Lösung von 3.3 g Triethanolamin in 10 ml Ethanol gleichzeitig zu und läßt 120 h unter Rückfluß kochen. Nach dem Abkühlen wird abgesaugt, das Filtrat eingeengt, der Rückstand in Diethylether aufgenommen, zweimal mit eiskalter 2 N Salzsäure ausgeschüttelt, mit Eiswasser neutral gewaschen, über Natriumsulfat getrocknet und eingeengt. Der Rückstand wird dann destilliert.

Ausbeute : 0.44 g (≙ 17 % d. Th.) 2-Cyan-1-ethoxyaziridin vom $Kp_{0,1}$ : 38-40 °C

## Beispiel 3

2-Cyan-1-ethoxyaziridin

Zu 1.93 g 2-Brom-3-ethoxyaminopropionitril (ölige Substanz) in 20 ml Methylenchlorid gibt man bei 0 °C 1.52 g Diazabicycloundecen und läßt die Lösung 48 h im Kühlschrank stehen. Dann wird eingeengt, der Rückstand mit Ether verrührt, filtriert und das Filtrat eingeengt. Den Rückstand nimmt man in wenig

eiskalter 2 N Salzsäure auf, extrahiert dreimal mit Ether, wäscht die Etherphase mit Wasser neutral, trocknet über Natriumsulfat und engt ein. Der Rückstand wird dann destilliert.

Ausbeute : 0.4 g (≙ 36 % d. Th.) 2-Cyan-1-ethoxyaziridin vom $Kp_{0,1}$ : 38-40 °C

Beispiel 4

1-(4-Chlorbenzyloxy)-2-cyanaziridin

Zu 4.7 g 2.3-Dibrompropionitril in 10 ml Ethanol tropft man bei 20 °C unter Rühren eine Lösung von 3.3 g Triethanolamin in 10 ml Ethanol zu. Nach 1 h saugt man ab, gibt zu dem Filtrat eine Lösung von 3.46 g 4-Chlorbenzyloxyamin in 20 ml Ethanol und läßt über Nacht bei Zimmertemperatur rühren. Anschließend wird eingeengt, der Rückstand in 50 ml Methylenchlorid aufgenommen und bei 0 °C mit 3.34 g Diazabicycloundecen versetzt. Die Lösung läßt man 48 h im Kühlschrank stehen, engt ein, verrührt den Rückstand mit Ether, saugt ab und engt das Filtrat ein. Den Rückstand gibt man auf eine Kieselgelsäule (100 g ; Fliesmittel : Ether/Ligroin i.V. 1/1). Das so erhaltene Produkt wird dann mit Ligroin verrührt und abgesaugt.

Ausbeute : 1.69 g (≙ 37 % d. Th.) 1-(4-Chlorbenzyloxy)-2-cyanaziridin vom Fp. 41-45 °C

In analoger Weise erhält man durch Umsetzung von 2.3-Dibrompropionitril mit

a) Phenethoxyamin das
2-Cyan-1-phenethoxyaziridin (ölige Substanz)

b) 2-Methylbenzyloxyamin das
2-Cyan-1-(2-methylbenzyloxy)-aziridin (Fp. 45-49 °C)

c) 3.4-Dimethoxy-benzyloxyamin das
2-Cyan-1-(3.4-dimethoxy-benzyloxy)-aziridin (Fp. 45-48 °C)

d) 2-Fluorbenzyloxyamin das
2-Cyan-1-(3-fluorbenzyloxy)-aziridin ($Kp_{0,1}$ : 138-140 °C)

Beispiel 5

1-Ethoxyaziridin-2-carboxamid

22 g 1-Ethoxyaziridin-2-carbonsäureethylester (Herstellung siehe nachstehend) löst man in 100 ml Ethanol/100 ml konz. Ammoniak und läßt 3 d bei Zimmertemperatur stehen. Die Lösung wird eingeengt, und über eine Kieselgelsäule gereinigt (200 g Kieselgel ; Elutionsmittel : Aceton/Toluol i.V. 1/1). Das Produkt kann aus Aceton umkristallisiert werden.

Ausbeute : 14.5 g (≙ 80 % d. Th.) 1-Ethoxyaziridin-2-carboxamid vom Fp : 50-54 °C

In analoger Weise erhält man

a) aus dem 1-Methoxyaziridin-2-carbonsäureethylester (s. Beispiel 5a) das
1-Methoxyaziridin-2-carboxamid ; Fp. 83-86 °C

b) aus dem 1-Isopropoxyaziridin-2-carbonsäureethylester (s. Beispiel 5b) das
1-Isopropoxyaziridin-2-carboxamid ; Fp. 118-120 °C

c) aus dem 1-n-Butoxyaziridin-2-carbonsäureethylester (s. Beispiel 5c) das
1-n-Butoxyaziridin-2-carboxamid ; Fp. 64-67 °C

d) aus dem 1-Benzyloxyaziridin-2-carbonsäureethylester (s. Beispiel 5d) das
1-Benzyloxyaziridin-2-carboxamid ; Fp. 87-90 °C

e) aus dem 1-Allyloxyaziridin-2-carbonsäureethylester (s. Beispiel 5e) das
1-Allyloxyaziridin-2-carboxamid ; Fp. 57-60 °C

Die als Ausgangsverbindungen eingesetzten Carbonsäureester werden wie folgt hergestellt :

1-Ethoxyaziridin-2-carbonsäureethylester

Zu 69.6 g 2.3-Dibrompropionsäureethylester in 140 ml Ethanol gibt man 40 g Triethanolamin in 40 ml Ethanol. Nach 1 h tropft man gleichzeitig eine Lösung von 16.3 g O-Ethylhydroxylamin in 30 ml Ethanol und 40 g Triethanolamin in 80 ml Ethanol zu und läßt 12 h bei Zimmertemperatur rühren. Der Niederschlag wird abgesaugt und das Filtrat 20 h unter Rückfluß erhitzt. Der Niederschlag wird wieder abgesaugt, das Filtrat eingeengt und über eine Kieselgelsäule gereinigt (100 g Kieselgel ; Elutionsmittel : Aceton/Toluol i.V. 1/1). Man erhält ein gelbes Öl, das destilliert wird.

Ausbeute : 25.5 g (≙ 60 % d. Th.) 1-Ethoxyaziridin-2-carbonsäureethylester vom $Kp_{0,1}$ : 38-40 °C

In analoger Weise erhält man aus 2,3-Dibrompropionsäureethylester und

a) O-Methylhydroxylamin den
1-Methoxyaziridin-2-carbonsäureethylester, $Kp_{0,2}$ : 42 °C

b) O-Isopropylhydroxylamin den
1-Isopropoxyaziridin-2-carbonsäureethylester

c) O-n-Butylhydroxylamin den
1-n-Butoxyaziridin-2-carbonsäureethylester

d) O-Benzylhydroxylamin den
1-Benzyloxyaziridin-2-carbonsäureethylester
e) O-Allylhydroxylamin den
1-Allyloxyaziridin-2-carbonsäureethylester

Beispiel 6

1-Allyloxy-2-cyanaziridin

Zu einer Suspension von 6.9 g 1-Allyloxy-2-aziridincarboxamid (Herstellung s. Beispiel 5e) in 110 ml Methylenchlorid gibt man unter Rühren bei Zimmertemperatur 25.6 g Triphenylphosphin, 14.8 g Tetrachlorkohlenstoff und 9.8 g Triethylamin. Man läßt 20 h rühren, engt am Rotavapor ein, nimmt den Rückstand in Diethylether auf, filtriert, engt das Filtrat ein und gibt den oeligen Rückstand auf eine Kieselgelsäule (100 g Kieselgel ; Elutionsmittel : Aceton/Toluol i.V. 1/1). Man erhält 2,6 g Rohprodukt, das anschließend destilliert wird.

Ausbeute : 1.9 g (≙ 32 % d. Th.) 1-Allyloxy-2-cyanaziridin vom $Kp_{0,1}$ : 58-59 °C
In analoger Weise erhält man aus
a) 1-Methoxyaziridin-2-carboxamid (s. Beispiel 5a) das
2-Cyan-1-methoxyaziridin ($Kp_{0,1}$ : 31-32 °C)
b) 1-Ethoxyaziridin-2-carboxamid (s. Beispiel 5) das
2-Cyan-1-ethoxyaziridin ($Kp_{0,1}$ : 38-40 °C)
c) 1-Isopropoxyaziridin-2-carboxamid (s. Beispiel 5b) das
2-Cyan-1-isopropoxyaziridin ($Kp_{0,1}$ : 28-29 °C)
d) 1-n-Butoxyaziridin-2-carboxamid (s. Beispiel 5c) das 1-n-Butoxy-2-cyanaziridin ($Kp_{0,1}$ : 83-84 °C)
e) 1-Benzyloxyaziridin-2-carboxamid (s. Beispiel 5d) das
1-Benzyloxy-2-cyanaziridin ($Kp_{0,1}$ : 113-115 °C)

Beispiel 7

Analog Beispiel 4 erhaelt man durch Umsetzung von 2.3-Dibrompropionitril mit
a) O-(2-Bromethyl)-hydroxylamin das
1-(2-Bromethoxy)-2-cyanaziridin (oelige Substanz)
b) O-(2-Hydroxyethyl)-hydroxylamin das
2-Cyan-1-(2-hydroxyethoxy)-aziridin (oelige Substanz)
c) O-(2-Tetrahydropyranyl)-hydroxylamin das
2-Cyan-1-(2-tetrahydropyranyloxy)-aziridin (oelige Substanz)
d) O-(2-Phenoxyethyl)-hydroxylamin das
2-Cyan-1-(2-phenoxyethoxy)-aziridin (oelige Substanz)
e) 1.2-Bis-(aminoxy)-ethan das
1.2-Bis-(2-cyan-1-aziridinyloxy)-ethan (oelige Substanz)
f) O-(2-Methyl-2-propenyl)-hydroxylamin das
2-Cyan-1-(2-methyl-2-propenyloxy)-aziridin $Kp_{0,1}$ : 65-67 °C
g) O-(3-propinyl)-hydroxylamin das
2-Cyan-1-(3-propinyloxy)-aziridin (oelige Substanz)
h) O-Cyclohexyl-hydroxylamin das
2-Cyan-1-cyclohexyloxy-aziridin (oelige Substanz)
i) O-(4-Cyanbenzyl)-hydroxylamin das
2-Cyan-1-(4-cyanbenzyloxy)-aziridin Fp : 48-51 °C (Ligroin)
k) O-(2-Pyridylmethyl)-hydroxylamin das
2-Cyan-1-(2-pyridylmethoxy)-aziridin (oelige Substanz)
l) O-(Ethoxycarbonylmethyl)-hydroxylamin den
2-Cyanaziridin-1-yl-oxyessigsaeureethylester (oelige Substanz)
m) O-(4-Methylbenzyl)-hydroxylamin das
2-Cyan-1-(4-methylbenzyloxy)-aziridin Fp : 20 °C
n) O-(3-ααα-Trifluormethylbenzyl)-hydroxylamin das
2-Cyan-1-(3-ααα-trifluormethylbenzyloxy)-aziridin oelige Substanz
o) O-(5-Chlor-2-methoxybenzyl)-hydroxylamin das
1-(5-Chlor-2-methoxybenzyloxy)-2-cyanaziridin Fp : 62-64 °C
p) O-(4-Methoxybenzyl)-hydroxylamin das
2-Cyan-1-(4-methoxybenzyloxy)-aziridin Fp : 44-46 °C
q) O-(2.4-Dichlorbenzyl)-hydroxylamin das
2-Cyan-1-(2.4-dichlorbenzyloxy)-aziridin Fp : 40-44 °C
r) O-(3.4-Dichlorbenzyl)-hydroxylamin das
2-Cyan-1-(3.4-dichlorbenzyloxy)-aziridin Fp : 48-50 °C
s) O-(Pyrimidin-2-ylmethyl)-hydroxylamin das

2-Cyan-1-(pyrimidin-2-ylmethoxy)-aziridin
t) O-(Pyrimidin-4-ylmethyl)-hydroxylamin das
2-Cyan-1-(pyrimidin-4-ylmethoxy)-aziridin

## Beispiel 8

Analog Beispiel 4 erhaelt man durch Umsetzung von 2.3-Dibrompropionitril mit O-(3-Fluorbenzyl)-hydroxylamin das 2-Cyan-1-(3-fluorbenzyloxy)-aziridin (siehe Beispiel 4d). Durch saeulenchromatographische Trennung an Kieselgel (Elutionsmittel : Ether/Ligroin i. V. 1 : 1) kann man die beiden Invertomeren trennen und erhaelt so

a) trans-2-Cyan-1-(3-fluorbenzyloxy)-aziridin (oelige Substanz) und
b) cis-2-Cyan-1-(3-fluorbenzyloxy)-aziridin (oelige Substanz)

Die beiden Invertomeren fallen im Verhaeltnis 5 : 1 (trans/cis) an. Sie werden durch die NMR-Spektren eindeutig charakterisiert.

## Beispiel 9

Die nachstehenden Beispiele betreffen pharmazeutische Zubereitungen, die Verbindungen der Formel Ia oder deren Salze enthalten.

### Beispiel A (Tabletten)

| | | | |
|---|---|---|---|
| Wirkstoff | | X mg | X = bis 40,0 mg |
| Lactose | ad | 60,0 mg | |
| Polyvinylpyrrolidon | | 2,0 mg | |
| mikrokrist. Cellulose | | 8,0 mg | |
| Natriumcarboxymethylamylopektin | | 4,0 mg | |
| Kieselsäure, hochdispers | | 0,5 mg | |
| Talkum | | 5,0 mg | |
| Magnesiumstearat | | 0,5 mg | |
| Endgewicht | | 80,0 mg | |

Für flüssige Wirkstoffe Dosierungen bis ca. 40 mg :

| | | | |
|---|---|---|---|
| Wirkstoff | | X mg | X = bis 40,0 mg |
| Kieselsäure, hochdispers | ad | 100,0 mg | |
| Lactose | | 135,0 mg | |
| Polyvinylpyrrolidon | | 10,0 mg | |
| mikrokrist. Cellulose | | 25,0 mg | |
| Natriumcarboxymethylamylopektin | | 10,0 mg | |
| Kieselsäure, hochdispers | | 2,0 mg | |
| Talkum | | 15,0 mg | |
| Magnesiumstearat | | 3,0 mg | |
| Endgewicht | | 300,0 mg | |

Wirkstoffe und Hilfsstoffe werden gemischt, gegebenenfalls granuliert und auf üblichen Maschinen zu Drageekernen verpreßt. Die Drageekerne werden in üblicher Weise mit einem magensaftresistenten, darmsaftlöslichen Film (z.B. einem anionischen Polymerisat aus Methacrylsäure und Methacrylsäuremethylester) überzogen.

| | | | |
|---|---|---|---|
| Wirkstoff | | X mg | X = bis 40,0 mg |
| Lactose | ad | 60,0 mg | |
| Magnesiumoxid | | 100,0 mg | |
| Polyvinylpyrrolidon | | 2,0 mg | |
| mikrokrist. Cellulose | | 8,0 mg | |
| Natriumcarboxymethylamylopektin | | 4,0 mg | |
| Kieselsäure, hochdispers | | 0,5 mg | |
| Talkum | | 5,0 mg | |
| Magnesiumstearat | | 0,5 mg | |
| Endgewicht | | 180,0 mg | |

Wirkstoff und Hilfsstoffe werden gemischt, ggf. granuliert und zu Tabletten verpreßt.

Beispiel B (Injektionslösung)

Als Präparationen für die Injektionsform, die 2-Cyan-1-methoxyaziridin enthält, können wäßrige Lösungen von Polyethylenglykol 400, Ethylenglykolmonoethylether, Ethanol sowie eine Lösung des Wirkstoffes in Miglyol 812-Neutralöl angegeben werden, wobei der letztere Hilfsstoff nur für intramuskuläre Applikation verwendet werden soll. Diese Zubereitungen wurden so konzipiert, daß pH-Wert, Pufferkapazität, Titrationsbasizität nicht stark von den physiologischen Werten abweichen.

Diese Injektionszubereitungen überstehen eine Sterilisation im Autoklaven 20 Minuten bei 121 °C ohne chemische Veränderungen.

Beispiel

| | | | | |
|---|---|---|---|---|
| 2-Cyan-1-methoxyaziridin | 40 mg | 40 mg | 40 mg | 40 mg |
| Polyethylenglykol 400 | 1 g | | | |
| Wasser | 3 g | 3 g | 4 g | |
| Ethylenglykolmonoethylether | | 2 g | 1 g | |
| Miglyol 812-Neutralöl | | | | 3 g |
| Ethanol | 1 g | | | |

Die Lösungsmittel werden zusammen mit dem Wirkstoff in einem Kessel gemischt. Die so erhaltene Lösung wird über Filterschichten Fibrafix AF steril filtriert. Die ersten 15 l sind Vorlauf und werden dem Ansatz zurückgeführt. Die Membranfiltration wird direkt an der Abfüllmaschine über Sartorius-Membranfilter, Porenweite 0,2 µm durchgeführt. Anschließend erfolgt die Abfüllung der Lösung in 5 ml-Ampullen. Die Lösung wird bei 121 °C 20 Minuten im Autoklaven sterilisiert.

Beispiel C (Weichgelatinekapseln)

Der Wirkstoff ist in organischen Verbindungen, wie Miglyol 812 (Triglycerid gesättigter Fettsäuren mit einer Kettenlänge C = 30), Gemischen von Ethanol in Wasser, Polyethylenglykol 400 in Wasser oder Ethylenglykolmonoethylether in Wasser löslich und kann in solchen Lösungen zu Weichgelatinekapseln verarbeitet werden. Auch läßt sich der Wirkstoff in Mischungen mit Wachs, Sojabohnenöl, Lecithin und hydrierten Fetten zu einer klassischen Weichgelatinerezeptur verarbeiten.

Beispiel

| | | | | | |
|---|---|---|---|---|---|
| 2-Cyan-1-methoxyaziridin | 40 mg | 40 mg | 40 mg | 40 mg | 40 mg |
| Bienenwachs | 20 mg | | | | |
| hydriertes Sojabohnenöl | 140 mg | | | | |
| Sojalecithin | 70 mg | | | | |
| Polyethylenglykol 400 | | 210 mg | | | 180 mg |
| Miglyol 812 | | 100 mg | 100 mg | 200 mg | 35 mg |
| Ethylenglykolmonoethylether | | | 210 mg | 50 mg | |
| Essigsäureethylester | | | | 43 mg | 85 mg |

Der Wirkstoff wird mit den entsprechenden Mengen der obengenannten Hilfsstoffe gemischt und auf einer Spezialmaschine zu Weichgelatinekapseln verschiedener Größen und Dosierungen verarbeitet.

Beispiel D (Tropfen und Saft)

| | | | | | | |
|---|---|---|---|---|---|---|
| 2-Cyan-1-methoxyaziridin | 2,5 ml | 2,5 ml | 2,5 ml | 2,5 ml | 2,5 ml | 2,5 ml |
| Polyethylenglykol 400 | — | 9,5 ml | 10,5 ml | 7,5 ml | — | 7,5 ml |
| Essigsäureethylester | — | 8,0 ml | 5,0 ml | — | 2,5 ml | — |
| Ethylenglykolmonoethylether | 12,0 ml | — | — | 9,0 ml | 3,0 ml | 3,0 ml |
| Miglyol 812 | 5,5 ml | — | 2,0 ml | 1,0 ml | 12,0 ml | |
| Wasser | | | | | | 7,0 ml |
| 2-Cyan-1-methoxyaziridin | | | 2,5 ml | 2,5 ml | 2,5 ml | 2,5 ml |
| Polyethylenglykol 400 | | | — | — | 12,0 ml | — |
| Ethylenglykolmonoethylether | | | 2,0 ml | — | — | 52,0 ml |
| Essigsäureethylester | | | — | — | — | 43,0 ml |
| Miglyol 812 | | | — | 154,0 ml | — | 80,0 ml |
| Wasser | | | 134,0 ml | | 143,0 ml | |

Der Wirkstoff wird mit den entsprechenden Mengen der obengenannten Hilfsstoffe gemischt. Die Mischung wird über Filterschichten Fibrafix AF steril sowie über Membranfilter mit einer Porenweite 0,2 μm filtriert. Es erfolgt Abfüllung 20 ml-Tropfenflaschen bzw. 200 ml-Saftflaschen.

Versuchsprotokoll

Bestimmung der Leukozyten nach einmaliger oraler Applikation an Rattan

Versuchsanordnung :

Es wurden weibliche erwachsene Sprague-Dawley-Ratten der Firma WIGA (Gassner, Sulzfeld) im Gewicht von 180-220 g verwendet. Die Tiere wurden bei konstanter Temperatur (23 ± 1 °C), konstanter Luftfeuchtigkeit (55 ± 5 %) und im 12-Stunden Tag/Nacht-Rythmus gehalten. Die Tiere erhielten Rattenpellets SNIFF der Firma Intermast, Soest, und Wasser ad libitum. Je 10 Ratten erhielten einmalig oral die zu untersuchenden Substanzen (gelöst in 10 ml 0,5 %iger Tyloselösung/kg Körpergewicht) mit Hilfe einer Schlundsonde appliziert. Zur Kontrolle wurden je 10 Tiere lediglich mit 10 ml 0,5 %iger Tyloselösung/kg Körpergewicht behandelt. Vor der Applikation wurden die Tiere nüchtern gesetzt und aus dem retroorbitalen Venenplexus mit Hilfe einer heparininisierten Stichkapillare (B 3095/2 der Firma Sherwood Med. Inc., St.-Louis) Blut entnommen und die Leukozyten mit Hilfe eines Coulter counters in bekannter Weise bestimmt.

Am 4. Tage wurde den Tieren wiederum Blut aus dem retroorbitalen Venenplexus entnommen und die Leukozyten gezählt. Aus den Einzelwerten wurden die Mittelwerte gebildet. Die Versuchsgruppen wurden nur ausgewertet, wenn die Kontrollgruppen keine physiologischen Schwankungen zeigten. Die Tabelle 1 zeigt die Werte im Vergleich zum 1-Carbamoyl-2-cyanaziridin (BA 1).

Die folgenden Daten zeigen, daß alle untersuchten Substanzen eine significante Steigerung der Leukozyten-Zahl bewirken und somit stark immunstimulierend sind.

TABELLE 1
Dosis : 200 mg/kg, per os

| Beispiel Nr. | Leukozyten in Tausend (x $10^2$) | |
|---|---|---|
| | 0 - Wert | Maximum(+ 4.Tag) |
| 1, 2, 3, 6b, | 8,04 | 15,68 |
| 1a, 6e | 7,29 | 16,53 |
| 1b, 6c | 9,1 | 21,5 |
| 4 | 7,03 | 12,45 |
| 4a | 8,23 | 12,88 |
| 4b | 7,16 | 10,99 |
| 4d | 6,84 | 10,84 |
| 6 | 7,48 | 11,59 |
| 6a | 5,89 | 19,29 |
| 7a | 6,6 | 12,9 |
| 7e | 5,6 | 11,3 |
| 7g | 6,6 | 10,3 |
| 7i | 6,7 | 11,4 |
| 1-Carbamoyl-2-cyanaziridin (Vergleichssubstanz) BA 1 | 8,9 | 9,5 |

Synergistischer Effekt von 2-Cyan-1-methoxy-aziridin (Beispiel 6a) mit Chloramphenicol

Versuchsanordnung :

Je 20 weibliche NMRI-Mäuse wurden mit E. coli (108) in einer Verdünnung von 1 : 60 in der Weise infiziert, daß 0,5 ml der Infektionslösung intraperitoneal gegeben wurden (Keimzahl : 5,4 × $10^6$ Keime/Tier). Es wurden insgesamt 4 Gruppen infiziert. Die 1. Gruppe wurde als Infektionskontrolle gewählt, die 2. Gruppe erhielt 20 mg/kg Chloramphenicol oral, die 3. Gruppe 10 mg/kg 2-Cyan-1-methoxy-aziridin

oral und die 4. Gruppe 20 mg/kg Chloramphenicol + 10 mg/kg 2-Cyan-1-methoxy-aziridin. Die Tiere wurden täglich bezüglich der Sterblichkeit beobachtet. Wie aus Tabelle 1 zu ersehen ist, stirbt in der Infektionskontrolle innerhalb von 4 Tagen alles, während in der mit Chloramphenicol behandelten Gruppe 13 von 20 überleben, in der mit Chloramphenicol + 2-Cyano-1-methoxy-aziridin 19 von 20 überleben. 2-Cyan-1-methoxy-aziridin allein hat lediglich einen geringen Effekt (2 von 20). Diese Sterblichkeitszahlen verschieben sich bis zum 14. Tag nicht mehr.

| | Überlebensrate | | |
|---|---|---|---|
| | 1. Tag | 4. Tag | 14. Tag |
| Chloramphenicol 20 mg/kg | 13/20 | 13/20 | 13/20 |
| 2-Cyan-1-methoxy-aziridin 10 mg/kg | 2/20 | 2/20 | 2/20 |
| Chloramphenicol (20 mg/kg) + 2-Cyan-1-methoxy-aziridin (10 mg/kg) | 20/20 | 19/20 | 19/20 |
| Kontrolle | 1/20 | 0/20 | 0/20 |

**Ansprüche**

1. N-substituierte 2-Cyanaziridine der allgemeinen Formel Ia

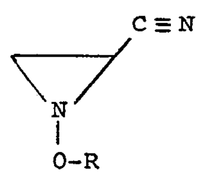

(Ia),

in der

R einen gesättigten $C_1$-$C_8$-Alkylrest, der ein- oder mehrfach durch Halogen, Hydroxy, Carboxyl, Nitril, Nitro oder einen $C_1$-$C_8$-Alkoxy-, $C_1$-$C_8$-Alkoxy-carbonyl-, Carbamoyl-, Formamido-, Acetamido-, Benzamido-, Dimethylamino-, Methylmercapto-, Methyl-sulfinyl- oder Methylsulfonyl-Rest oder einen $C_3$-$C_{10}$-Cycloalkyl- oder Cycloalkenyl-Rest oder einen Phenoxy-Rest oder einen Phenyl-Rest substituiert sein kann, welcher wiederum gegebenenfalls ein- oder mehrfach durch Halogen, Hydroxy, Nitro, Trifluormethyl, Nitril, Methylmercapto oder einen $C_1$-$C_8$-Alkyl- oder Alkoxy-Rest substituiert ist,

einen ein- oder mehrfach ungesättigten Alkenyl- oder Alkinyl-Rest mit 3-8 C-Atomen, einen $C_3$-$C_{10}$-Cycloalkyl- oder Cycloalkenyl-Rest, einen Cinnamyl-, Norbornyl- oder Adamantyl-Rest, einen Phenyl- oder Naphthyl-Rest oder einen Tetrahydrofurfuryl-, Tetrahydropyranyl-, Acetoxyäthyl-, Morpholino-ethyl-, N-Acetyl-piperidin-4-ylmethyl-, Thianyl-, Pyridinylmethyl-, Furfuryl-, Thenyl-, Pyrimidinyl-methyl-, 2-Oxazolidinon-5-ylmethyl- oder 2-Cyan-1-aziridinyloxyethyl-Rest

bedeutet, sowie deren pharmakologisch verträglichen Salze.

2. Verbindungen gemäß Anspruch 1, wobei R Methyl, Ethyl, Isopropyl, 2-Bromethyl, Allyl, 3-Propinyl, Benzyl, 4-Chlorbenzyl, 2-Methylbenzyl, 3-Fluorbenzyl, 5-Chlor-2-methoxybenzyl, 4-Cyanbenzyl, 2-Phenethyl oder 2-Cyan-1-aziridinyl-oxyethyl bedeutet.

3. N-substituierte Aziridin-2-carbonsäureamide der Formel Ib

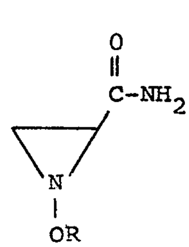

(Ib),

in der

R die in Anspruch 1 angegebene Bedeutung hat.

4. Verfahren zur Herstellung von N-substituierten Aziridin-2-carbonsäurederivaten der allgemeinen Formel I

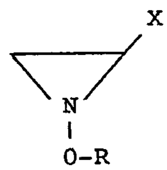

(I)

in der

X eine Nitril- oder Carbamoyl-Gruppe und

R einen gesättigten $C_1$-$C_8$-Alkylrest, der ein- oder mehrfach durch Halogen, Hydroxy, Carboxyl, Nitril, Nitro oder einen $C_1$-$C_8$-Alkoxy-, $C_1$-$C_8$-Alkoxy-carbonyl-, Carbamoyl-, Formamido-, Acetamido-, Benzamido-, Dimethylamino-, Methylmercapto-, Methylsulfinyl- oder Methylsulfonyl-Rest oder einen $C_3$-$C_{10}$-Cycloalkyl- oder Cycloalkenyl-Rest oder einen Phenoxy-Rest oder einen Phenyl-Rest substituiert sein kann, welcher wiederum gegebenenfalls ein- oder mehrfach durch Halogen, Hydroxy, Nitro, Trifluormethyl, Nitril, Methylmercapto oder einen $C_1$-$C_8$-Alkoxy-Rest substituiert ist,

einen ein- oder mehrfach ungesättigten Alkenyl- oder Alkinyl-Rest mit 3-8 C-Atomen, wobei das ungesättigte C-Atom nicht mit dem Sauerstoffatom verbunden ist, einen $C_3$-$C_{10}$-Cycloalkyl- oder Cycloalkenyl-Rest, einen Cinnamyl-, Norbornyl- oder Adamantyl-Rest, einen Phenyl- oder Naphthyl-Rest oder einen Tetrahydrofurfuryl-, Tetrahydropyranyl-, Acetoxyäthyl-, Morpholinoethyl-, N-Acetyl-piperidin-4-ylmethyl-, Thianyl-, Pyridinylmethyl-, Furfuryl-, Thenyl-, Pyrimidinylmethyl-, 2-Oxazolidinon-5-yl-methyl- oder 2-Cyan-1-aziridinyloxy-ethyl-Rest

bedeuten, sowie deren pharmakologisch verträglichen Salze, dadurch gekennzeichnet, daß man in an sich bekannter Weise

a) eine Verbindung der allgemeinen Formel II

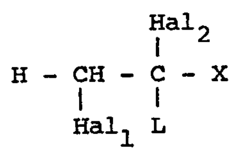

(II)

in der

X die oben angegebene Bedeutung hat, $Hal_1$ und $Hal_2$ Chlor oder Brom sind, L Wasserstoff bedeutet, wobei $Hal_1$ und L zusammen auch ein Valenzstrich sein können,

mit einem Hydroxylaminderivat der allgemeinen Formel III

$$R—O—NH_2 \qquad (III)$$

in der

R die oben angegebene Bedeutung hat,

umsetzt oder

b) eine Verbindung der allgemeinen Formel IVa oder IVb bzw. deren Salze

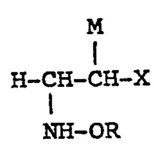

(IVa)

bzw.

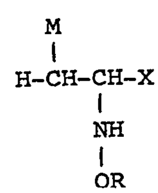

(IVb),

in denen

R und X die oben angegebenen Bedeutungen haben und M Chlor, Brom oder die Gruppe A-Z bedeuten soll, wobei A Sauerstoff oder Schwefel und Z Wasserstoff oder eine zusammen mit Sauerstoff bzw. Schwefel leicht eliminierbare Gruppierung darstellen soll,
mit einem M-H-abspaltenden Reagenz behandelt
oder
c) eine Verbindung der allgemeinen Formel V

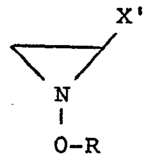
(V)

in der
R die oben angegebene Bedeutung hat und X′ die —CH = $NOR_2$-Gruppe darstellt, wobei $R_2$ Wasserstoff oder eine Alkylgruppe vorstellt,
mit einem wasser- bzw. alkoholabspaltenden Reagenz behandelt
oder
d) ein Oxazolidinon der allgemeinen Formel VIa oder VIb

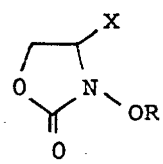
bzw.
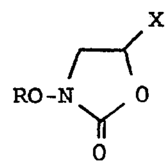

(VIa) (VIb),

in denen
R und X die oben angegebenen Bedeutungen haben,
einer Thermolyse unterwirft
oder
e) eine Verbindung der allgemeinen Formel VIIa oder VIIb

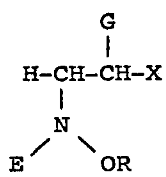
bzw.
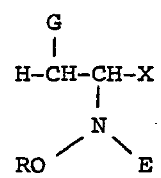

(VIIa) (VIIb),

in denen
R und X die oben angegebenen Bedeutungen haben und G Wasserstoff, Chlor oder Brom und E Chlor, Brom, eine Trialkylaminogruppe oder einen Arylsulfonsäureresterrest bedeuten,
mit einem E-G-apspaltenden Reagenz behandelt
oder
f) ein Epoxyd der allgemeinen Formel VIII

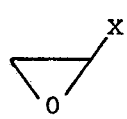
(VIII)

in der
X die oben angegebene Bedeutung hat,
mit einem Hydroxylaminderivat der allgemeinen Formel III umsetzt,

anschließend gewünschtenfalls eine nach einem der vorstehenden Verfahren erhaltene Verbindung der allgemeinen Formel I mit X = Carbamoyl in die entsprechende Nitril-Verbindung überführt oder einen Substituenten R in einen anderen durch den Anspruch definierten Substituenten R umwandelt und

gegebenenfalls eine so erhaltene Verbindung der allgemeinen Formel Ia in ein pharmakologisch verträgliches Salz überführt.

5. Verbindungen gemäß Ansprüche 1 und 2 zur Verwendung bei der Bekämpfung von mit einer Schwächung des Immunsystems verbundenen Krankheiten.

6. Arzneimittel, enthaltend eine Verbindung der im Anspruch 1 angegebenen Formel Ia sowie übliche Träger- und Hilfsstoffe.

7. Arzneimittel gemäß Anspruch 6, dadurch gekennzeichnet, daß es zusätzlich ein Chemotherapeuticum enthält.

8. Arzneimittel gemäß Anspruch 7, dadurch gekennzeichnet, daß als Chemotherapeuticum Penicilline, Cephalosporine, Sulfonamide, Aminoglykosid-Antibiotika oder Tetracycline verwendet werden.

9. Arzneimittel gemäß Anspruch 7, dadurch gekennzeichnet, daß als Chemotherapeuticum Chloramphenicol verwendet wird.

**Claims**

1. N-Substituted 2-cyanoaziridines of the general formula Ia

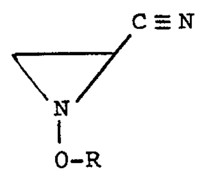

(Ia)

in which R signifies a saturated $C_1$-$C_8$ alkyl radical which can be substituted one or more times by halogen, hydroxyl, carboxyl, nitrile, nitro or a $C_1$-$C_8$-alkoxy, $C_1$-$C_8$-alkoxycarbonyl, carbamoyl, formamido, acetamido, benzamido, dimethylamino, methylmercapto, methylsulphinyl or methylsulphonyl radical or a $C_3$-$C_{10}$ cycloalkyl or cycloalkenyl radical or a phenoxy radical or a phenyl radical which, in turn, is possibly substituted one or more times by halogen, hydroxyl, nitro, trifluoromethyl, nitrile, methylmercapto or a $C_1$-$C_8$-alkyl or alkoxy radical ; a mono- or poly-unsaturated alkenyl or alkynyl radical with 3-8 C-atoms, a $C_3$-$C_{10}$-cycloalkyl or cycloalkenyl radical, a cinnamyl, norbornyl or adamantyl radical, a phenyl or naphthyl radical or a tetrahydrofurfuryl, tetrahydropyranyl, acetoxyethyl, morpholinoethyl, N-acetyl-piperidin-4-ylmethyl, thianyl, pyridinylmethyl, furfuryl, thenyl, pyrimidinylmethyl, 2-oxazolidinon-5-ylmethyl or 2-cyano-1-aziridinyloxyethyl radical ; as well as their pharmacologically acceptable salts.

2. Compounds according to claim 1, whereby R signifies methyl, ethyl, isopropyl, 2-bromoethyl, allyl, 3-propynyl, benzyl, 4-chlorobenzyl, 2-methylbenzyl, 3-fluorobenzyl, 5-chloro-2-methoxybenzyl, 4-cyanobenzyl, 2-phenethyl or 2-cyano-1-aziridinyloxy-ethyl.

3. N-Substituted aziridine-2-carboxylic acid amides of the formula Ib

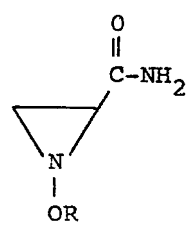

(Ib)

in which R has the meaning given in claim 1.

4. Process for the preparation of N-substituted aziridine-2-carboxylic acid derivatives of the general formula I

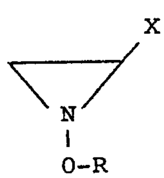

(I)

in which X signifies a nitrile or carbamoyl group and R a saturated $C_1$-$C_8$-alkyl radical which can be substituted one or more times by halogen, hydroxyl carboxyl, nitrile, nitro or a $C_1$-$C_8$-alkoxy, $C_1$-$C_8$-alkoxycarbonyl, carbamoyl, formamido, acetamido, benzamido, diethylamino, methylmercapto, methylsulphinyl or methylsulphonyl radical or a $C_3$-$C_{10}$-cycloalkyl or cycloalkenyl radical or a phenoxy radical or a phenyl radical which, in turn, is optionally substituted one or more times by halogen, hydroxyl, nitro, trifluoromethyl, nitrile, methylmercapto or a $C_1$-$C_8$-alkyl or alkoxy radical ; a mono- or polyunsaturated alkenyl or alkynyl radical with 3-8 C-atoms, whereby the unsaturated C-atom is not connected with the oxygen atom, a $C_3$-$C_{10}$-cycloalkyl or cycloalkenyl radical, a cinnamyl, norbornyl or adamantyl radical, a phenyl or naphthyl radical or a tetrahydrofurfuryl, tetrahydropyranyl, acetoxyethyl, morpholinoethyl, N-acetylpiperidin-4-ylmethyl, thianyl, pyridinylmethyl, furfuryl, thenyl, pyrimidinylmethyl, 2-oxazolidinon-5-ylmethyl or 2-cyano-1-aziridinyloxyethyl radical ; as well as of their pharmacologically acceptable salts, characterised in that, in per se known manner, one

a) reacts a compound of the general formula II

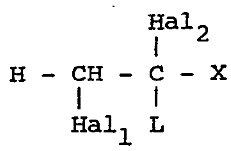

(II)

in which X has the above-given meaning, $Hal_1$ and $Hal_2$ are chlorine or bromine, L signifies hydrogen, whereby $Hal_1$ and L together can also be a valency bond, with a hydroxylamine derivative of the general formula III

$$R—O—NH_2 \qquad (III)$$

in which R has the above-given meaning ; or

b) treats a compound of the general formula IVa or IVb or their salts

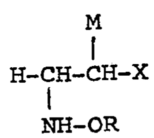

(IVa)

or

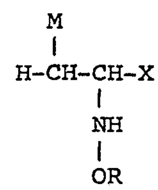

(IVb)

in which R and X have the above-given meanings and M is to be chlorine, bromine or the group -A-Z, whereby A is to represent oxygen or sulphur and Z hydrogen or a grouping which, together with oxygen or sulphur, is easily eliminated, with a reagent splitting off M-H ; or

c) treats a compound of the general formula V

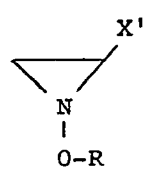

(V)

in which R has the above-given meaning and X' represents the $—CH=NOR_2$ group, whereby $R_2$ represents hydrogen or an alkyl group, with a reagent splitting off water or an alcohol ; or

d) subjects an oxazolidinone of the general formula VIa or VIb

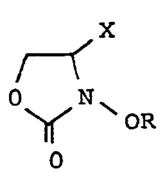

(VIa) or

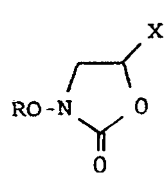

(VIb)

in which R and X have the above-given meanings, to a thermolysis ; or

e) treats a compound of the general formula VIIa or VIIb

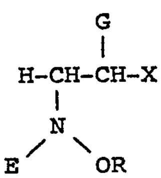 or 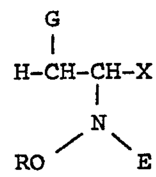

(VIIa) (VIIb)

in which R and X have the above-given meanings and G signifies hydrogen, chlorine or bromine and E chlorine, bromine, a trialkylamino group or an arylsulphonic acid ester residue, with a reagent splitting off E-G ; or

f) reacts an epoxide of the general formula VIII

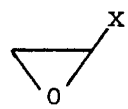

(VIII)

in which X has the above-given meaning, with a hydroxylamine derivative of general formula III ; subsequently, if desired, converts a compound of general formula I obtained according to one of the above processes with X = carbamoyl into the corresponding nitrile compound or changes a substituent R into another substituent R defined by the claim and, if desired, converts a so obtained compound of the general formula Ia into a pharmacologically acceptable salt.

5. Compounds according to claims 1 and 2 for use in the combating of diseases involving a weakening of the immune system.

6. Medicament containing a compound of the formula Ia given in claim 1, as well as conventional carrier and adjuvant materials.

7. Medicament according to claim 6, characterised in that it additionally contains a chemotherapeutic agent.

8. Medicament according to claim 7, characterised in that as chemotherapeutic agent there are used penicillins, cephalosporins, sulphonamides, aminoglycoside antibiotics or tetracyclines.

9. Medicaments according to claim 7, characterised in that as chemotherapeutic agent there is used chloramphenicol.

**Revendications**

1. Cyanaziridines N-substituées de formule générale Ia

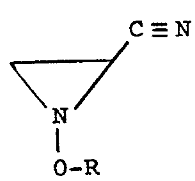

(Ia)

dans laquelle

R est un reste alkyle saturé $C_1$-$C_8$, pouvant être substitué une ou plusieurs fois par de l'halogène, un groupe hydroxy, carboxyle, nitrile ou nitro, ou par un reste alcoxy $C_1$-$C_8$, alcoxycarbonyle $C_1$-$C_8$, carbamoyle, formamido, acétamido, benzamido, diméthylamino, méthylmercapto, méthylsulfinyle ou méthylsulfonyle ou par un reste cyclo-alkyle $C_3$-$C_{10}$ ou cyclo-alcényle ou par un reste phénoxy ou par un reste phényle, ce substituant pouvant être substitué à son tour une ou plusieurs fois par de l'halogène, un groupe hydroxy, nitro, trifluorométhyle, nitrile, méthylmercapto ou par un reste alkyle $C_1$-$C_8$ ou alcoxy,

ou un reste alcényle ou alcinyle une ou plusieurs fois insaturé ayant de 3 à 8 atomes de carbone, un reste cyclo-alkyle $C_3$-$C_{10}$ ou cyclo-alcényle, un reste cinnamyle, norbornyle ou adamantyle, un reste phényle ou naphtyle ou un reste tétrahydrofurfuryle, tétrahydropyranyle, acétoxyéthyle, morpholino-éthyle, N-acétyl-pipéridine 4-ylméthyle, thianyle, pyridinylméthyle, furfuryle, thényle, pyrimidinyl-méthyle,

2-oxazolidinone 5-ylméthyle ou 2-cyano 1-aziridinyloxyéthyle, ainsi que leurs sels pharmacologiquement tolérables.

2. Composés selon la revendication 1, où R est méthyle, éthyle, isopropyle, 2-bromo-éthyle, allyle, 3-propinyle, benzyle, 4-chlorobenzyle, 2-méthylbenzyle, 3-fluorobenzyle, 5-chloro 2-méthoxybenzyle, 4-cyanobenzyle, 2-phénéthyle ou 2-cyano 1-aziridinyl-oxyéthyle.

3. Amides N-substitués de l'acide aziridine 2-carboxylique de formule Ib

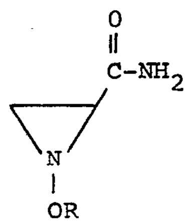

(Ib)

dans laquelle

R a la signification indiquée dans la revendication 1.

4. Procédé pour la préparation de dérivés N-substitués de l'acide aziridine 2-carboxylique de formule générale I

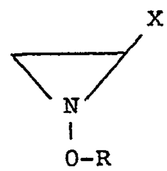

(I)

dans laquelle

X est un groupe nitrile ou carbamoyle et

R est un reste alkyle saturé $C_1$-$C_8$, pouvant être substitué une ou plusieurs fois par de l'halogène, un groupe hydroxy, carboxyle, nitrile ou nitro, ou par un reste alcoxy $C_1$-$C_8$, alcoxycarbonyle $C_1$-$C_8$, carbamoyle, formamido, acétamido, benzamido, diméthylamino, méthylmercapto, méthylsulfinyle ou méthylsulfonyle ou par un reste cyclo-alkyle $C_3$-$C_{10}$ ou cyclo-alcényle ou par un reste phénoxy ou par un reste phényle, ce substituant pouvant être substitué à son tour une ou plusieurs fois par de l'halogène, un groupe hydroxy, nitro, trifluorométhyle, nitrile, méthyl-mercapto ou par un reste alkyle $C_1$-$C_8$ ou alcoxy,

ou un reste alcényle ou alcinyle une ou plusieurs fois insaturé ayant de 3 à 8 atomes de carbone, un reste cyclo-alkyle $C_3$-$C_{10}$ ou cyclo-alcényle, un reste cinnamyle, norbornyle ou adamantyle, un reste phényle ou naphtyle ou un reste tétrahydrofurfuryle, tétrahydropyranyle, acétoxyéthyle, morpholino-éthyle, N-acétyl-pipéridine 4-ylméthyle, thianyle, pyridinylméthyle, furfuryle, thényle, pyrimidinyl-méthyle, 2-oxazolidinone 5-ylméthyle ou 2-cyano 1-aziridinyloxyéthyle,

ainsi que leurs sels pharmacologiquement tolérables, caractérisé en ce que de façon en soi connue

a) on fait réagir un composé de formule générale II

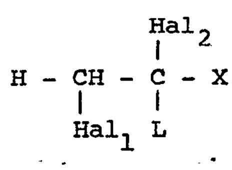

(II)

dans laquelle

X a la signification donnée ci-dessus, $Hal_1$ et $Hal_2$ sont du chlore ou du brome, L est de l'hydrogène, $Hal_1$ et L pouvant aussi former ensemble un trait de valence,

avec un dérivé d'hydroxylamine de formule générale III

$$R\text{—}O\text{—}NH_2 \qquad \text{(III)}$$

dans laquelle

R a la signification donnée ci-dessus,

ou,

b) on traite un composé de formule générale IVa ou IVb ou ses sels

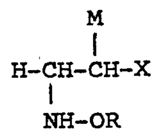 ou 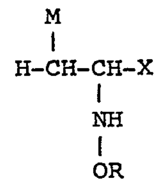

(IVa) (IVb)

dans lesquelles

R et X ont la signification indiquée ci-dessus et M est du chlore, du brome ou le groupe A-Z ou A est de l'oxygène ou du soufre et Z de l'hydrogène, ou représente ensemble avec l'oxygène ou le soufre un groupe facilement éliminable,

avec un réactif éliminant le groupe M-H,

ou,

c) on traite un composé de formule générale V

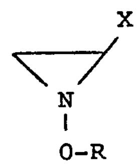 (V)

dans laquelle

R a la signification indiquée ci-dessus et X' est le groupe —CH = $NOR_2$ où $R_2$ est de l'hydrogène ou un groupe alkyle avec un réactif éliminant de l'eau ou de l'alcool,

ou,

d) on soumet une oxazolidinone de formule générale VIa ou VIb

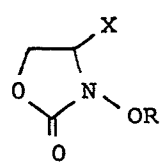 ou 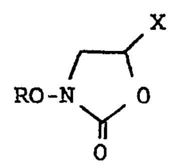

(VIa) (VIb)

dans lesquelles

R et X ont la signification donnée ci-dessus à une thermolyse

ou

e) on traite un composé de formule générale VIIa ou VIIb

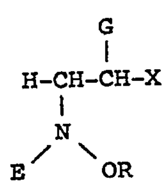 ou 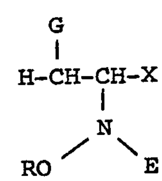

(VIIa) (VIIb)

dans lesquelles

R et X ont la signification donnée ci-dessus et G est de l'hydrogène, du chlore ou du brome et E du chlore, du brome, un groupe trialkylamino ou un reste d'un ester d'un acide arylsulfonique,

avec un réactif éliminant le groupe E-G

ou

f) on fait réagir un époxyde de formule générale VIII

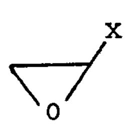 (VIII)

dans laquelle

X a la signification indiquée ci-dessus, avec un dérivé d'hydroxylamine de formule générale III, et en ce qu'ensuite on transforme si l'on désire un composé obtenu selon le procédé précédent, de formule générale I, avec X = carbamoyle en un dérivé nitrilé correspondant, ou on transforme un substituant R en un autre substituant défini dans la revendication principale et

en ce qu'éventuellement on transforme un composé ainsi obtenu de formule générale Ia en un sel pharmacologiquement tolérable.

5. Composés selon les revendications 1 et 2 pour l'utilisation dans la lutte contre les maladies ayant un rapport avec un affaiblissement du système immunisant.

6. Médicament contenant un composé répondant à la formule Ia de la revendication 1, ainsi que les substances de support et auxiliaires habituelles.

7. Médicament selon la revendication 6, caractérisé en ce qu'il comprend additionnellement une substance pour chimiothérapie.

8. Médicament selon la revendication 7 caractérisé en ce que la substance pour chimiothérapie est de la pénicilline, de la céphalosporine, du sulfonamide, un antibiotique du type aminoglycoside ou de la tétracycline.

9. Médicament selon la revendication 7 caractérisé en ce que la substance pour chimiothérapie est du chloramphénicol.